# EUROPEAN PATENT APPLICATION

(11) **EP 2 815 784 A1**
(43) Date of publication of application: **24.12.2014**
(21) Application number: 13749343.3
(22) Date of filing: 14.02.2013
(51) Int. Cl.: A61M 37/00, A61L 31/00

(54) **MICRONEEDLE OF SHORT-TIME DISSOLUTION TYPE**

(30) Priority: 17.02.2012 JP 2012048530
(71) Applicant: Cosmed Pharmaceutical Co., Ltd., Kyoto-shi, Kyoto 601-8014 (JP)
(72) Inventor: QUAN, Ying-shu, Kyoto-shi Kyoto 601-8014 (JP); KAMIYAMA, Fumio, Kyoto-shi Kyoto 601-8014 (JP)
(74) Representative: Ter Meer Steinmeister & Partner
(86) International application number: PCT/JP2013/053556
(87) International publication number: WO 2013/122160

(57) **Abstract**

According to the present invention, a microneedle material capable of dissolving in a short-time and being absorbed quickly in order to shorten the application time of the microneedle is provided.

A mixture of a water-soluble polymer and one or more saccharides selected from monosaccharides and disaccharides is used as the microneedle material. A systhetic polymer, protein, polysaccharide or a mixture thereof can be preferably used as the water-soluble polymer. Glucose, fructose, sucrose, lactose, trehalose, or a mixture thereof can be preferably used as the saccharides.

## Description

### Technical Field

The present invention relates to a material for dissolving a microneedle for giving modification effects and/or functional effects on a skin surface and/or stratum corneum in a short time.

As a method of administering a valuable material to a human body, oral administration and transdermal administration are often used. Injection is a typical transdermal administration method. However, injection is a procedure which takes time and labor of specialists such as physicians and nurses, is painful and is likely to cause an infection of AIDS, hepatitis B etc. so that many people do not welcome the procedure. In contrast, a transdermal administration method without pain using a microneedle array has been recently attracting attention (Patent Document 1, Non-patent Document 1).

Herein, the valuable material means a substance such as a drug and a cosmetic which shows preferable effects by being administered to a human body.

In transdermal administration of a valuable material, stratum corneum works as a barrier to permeation of the valuable material so that only applying the valuable material on a skin surface cannot cause enough permeability. In contrast, perforation of corneum by using a minute needle, i.e. a microneedle can remarkably improve efficiency in administration of a valuable material compared to that in the application method. It is a microneedle array in which a large number of the microneedles are integrated on a substrate. In addition, a product in which various tapes such as an adhesive tape for adhering the microneedle array to a skin and a cover sheet for maintaining an aseptic state until its use are added to the microneedle array in order to facilitate its use is called a microneedle patch.

Herein, the tape means a film, or a cloth or paper to which an adhesive is applied.

When a microneedle is produced by using a substance such as a water-soluble polymer which dissolves in a body and disappears by metabolism as a material, an accident is not caused even if the microneedle is broken and remains in a skin. Furthermore, if a valuable material is contained in the water-soluble polymer, the valuable material can be easily administered into and under the skin by dissolving the inserted microneedle in the body (Patent Document 2).

Particularly, when a microneedle comprising a biosoluble polymer material such as hyaluronic acid and collagen is inserted into a skin, moisture in the skin diffuses in the microneedle, and a needle portion inserted into the skin swells and then dissolves. As a result, hyaluronic acid or collagen diffuses in the skin to express an antiwrinkle action, or otherwise a valuable material or a valuable substance previously dissolved in the needle portion diffuses in the skin (Patent Documents 3 and 4).

It takes an average of 3-10 hours for a microneedle comprising, as a main material, a water-soluble polymer such as hyaluronic acid to swell and dissolve in a skin depending on a water content in the skin. For this reason, in a case that the microneedle array is applied to a facial skin, it is often applied before bedtime, and it was difficult to apply it to a skin at a dermatology office or a beauty salon. Also in a case that it was used for medical application, a long-time application was required for dissolving the microneedle inserted into the skin, and shortening of the application time was required also from the aspect of improvement of patient QOL. For microneedle administration of insulin to a diabetes patient, the blood glucose level should be rapidly lowered in some cases. Also in a case of administration of a vaccine microneedle, if its application to a skin is completed in about 30 minutes, its convenience is considerably sufficient.

Herein, the application of the microneedle array to the skin means that the microneedles are inserted into the skin and held as they are for an adequate period.

A cosmetic using a microneedle was commercially produced by CosMED Pharmaceutical Co. Ltd for the first time in the world, and released on October 2008 (Non-Patent Document 1). Since this microneedle took a lot of time to swell and dissolve, demanders had strongly desired improvements for accelerating its solution rate of the microneedle and enabling rapid application.

Valuable materials to be contained in the microneedle array are often extremely expensive or can be obtained only in minute amounts. When such an expensive and precious valuable material is contained in a material to produce a microneedle array by a conventional method, the valuable material is contained not only in the microneedle portion but also in its substrate portion. When this microneedle array is inserted into a skin, a valuable material contained in the microneedle portion is incorporated and diffused in a body, but the valuable material remaining in the substrate portion is discarded without utilization, resulting in low usage efficiency of the expensive valuable material. For avoiding loss of the expensive valuable material, the valuable material should be hold only in the tip of the microneedle.

For holding the valuable material only in the tip of the microneedle, a method that the microneedle tip is soaked in an aqueous solution of the valuable material and it is adhered only to the tip may be exemplified (Patent Document 5). However, a problem that, in the conventional method for merely adhering the valuable material to the tip, when the microneedle is inserted into a skin, an adhered part is broken and removed, resulting in insufficient uptake of the valuable material, should be solved.

### Prior Art Documents

### Patent Documents

[Patent Document 1] JP-2002517300W
[Patent Document 2] JP-2003238347A
[Patent Document 3] JP-2009273872A
[Patent Document 4] JP-2010029634A
[Patent Document 5] WO/2008/139648

### Non-Patent Documents

[Non-Patent Document 1] YonSuk KA, Fumio KAMIYAMA "The Course of Productization of Microneedle", The Academy of Pharmaceutical Science and Technology, Japan; September 2009, Vol. 69, 4th issue, p.272-276

### Summary of the Invention

### Technical Problem

The problem to be solved by the present invention is to provide a microneedle which rapidly dissolves and is rapidly absorbed and to solve the problem in the prior art. That is, a design of a microneedle patch is considered, and a microneedle material which can reduce a time to swell and dissolve the microneedle array is provided.

### Solution to Problem

The short-time soluble microneedle related to the present invention made for solving the problems is characterized in that it comprises, as a microneedle material, a mixture of a water-soluble polymer and one or more saccharides selected from monosaccharides and disaccharides.

If a microneedle is produced by a material in which the monosaccharides and disaccharides were added to the water-soluble polymer so that their content is 5-40 wt% with respect to the total weight of the material of the mixture, a dissolution time of the microneedle in a skin can be within 30 minutes. If the mixing amount of the monosaccharides and disaccharides is less than 5 wt%, the effect to reduce the dissolution time is low. If the mixing amount is more than 40 wt%, a mechanical strength of the microneedle is lowered, the microneedle is difficult to produce and to insert into a skin in its use. For producing the microneedle, a method in which a material aqueous solution is casted into a mold at normal temperature, dried, and then pulled out from the mold is usually used, but if the mechanical strength is lowered, it is hardly pulled out from the mold. In addition, the microneedle having low mechanical strength cannot be inserted because it is broken during insertion into the skin.

As a water-soluble polymer, synthetic polymer such as polyvinylpyrrolidone or polyvinyl alcohol; protein such as collagen (or hydrolyzed collagen) or gelatin; polysaccharide such as hyaluronic acid, dextrin, dextran, proteoglycans or sodium chondroitin sulfate; carboxymethyl cellulose hydroxyethyl cellulose, etc. are preferable.

As monosaccharides and disaccharides to be mixed with a water-soluble polymer, glucose, fructose, sucrose, lactose, trehalose, or a mixture thereof can be preferably used. Among them, glucose is the most preferable.

When the microneedle array is applied, the microneedle portion is inserted into the skin, and thus if the material of the microneedle portion is a mixture of a water-soluble polymer and one or more saccharides selected from monosaccharides and disaccharides, the substrate portion of the microneedle array may be made from another material.

In addition, the microneedle may be impregnated with a valuable material of a cosmetic ingredient or a medicinal component, particularly a polymer medicinal component, etc. A content of the valuable material is preferably in a range of 0.001 % to 20% with respect to the total weight. Examples of the cosmetic ingredient include whitening ingredient such as ascorbyl palmitate, kojic acid, rucinol, tranexamic acid, oil licorice extract, vitamin A derivative, placenta extract or adenosine sulfate Ma; antiwrinkle ingredient such as retinol, retinoic acid, retinol acetate, retinol palmitate, EGF, cell culture extract or adenosine; blood circulation promoting ingredient such as α -tocopherol, tocopherol acetate, capsin or nonylic acid vanillylamide; antiobesity ingredient such as raspberry keton, evening primrose extract or seaweed extract; antimicrobial ingredient such as isopropyl methylphenol, photosensitizer or zinc oxide; vitamins such as vitamin D2, vitamin D3 or vitamin K, etc.

Examples of the polymer medicinal component include physiologically active polypeptides and derivatives thereof, nucleic acid, oligonucleotide, various antigen proteins for vaccine use, bacteria, fragment of virus, etc.

Examples of the polypeptides and the derivatives thereof include calcitonin, adrenocorticotropic hormone, parathyroid hormone (PTH), hPTH (1→34), insulin, exendin and derivatives threrof, secretin, oxytocin, angiotensin, β-endorphin, glucagon, vasopressin, somatostatin, gastrin, luteinizing hormone-releasing hormone, enkephalin, neurotensin, atrial natriuretic peptide, growth hormone, growth hormone-releasing hormone, FGF, bradykinin, substance P, dynorphin, erythropoietin, thyroid stimulating hormone, prolactin, interferons, interleukins, G-CSF, glutathione peroxidase, superoxide dismutase, desmopressin, somatomedin, endothelin, salts thereof, etc. Examples of the antigen proteins include influenza virus antigen, HBs surface antigen, HBe antigen, etc.

In relation to a general valuable material, a microneedle array containing a valuable material can be produced, by producing it with a usual method in which a valuable material is dissolved in an aqueous solution of a microneedle material, the aqueous solution is casted into a mold, dried, and then pulled out from the mold.

When the valuable material is expensive, the valuable material should be contained only in the microneedle tip, because containing of the valuable material in the whole microneedle array causes loss of the valuable material contained in the substrate portion of the microneedle array. However, only soaking of the microneedle tip in the aqueous solution of the valuable material for adhesion is not enough to incorporate the valuable material, because the valuable material-adhered portion is broken or removed when inserting the microneedles into the skin.

When the valuable material is water-soluble, the expensive valuable material can be efficiently administered according to the following procedures. The microneedles are produced by using a mixture of water-soluble polymers and saccharides (monosaccharides or disaccharides) as a material. The valuable material is dissolved in the mixture solution of the water-soluble polymers and the saccharides (monosaccharides or disaccharides). The microneedle tips are impregnated with the valuable material solution, the valuable material is adhered to the microneedle tips, and then dried to produce valuable material-adhered microneedles. In this way, the valuable material is incorporated in the microneedle tips together with the mixture of the water-soluble polymers and the saccharides as the microneedle material. In this way, the microneedle is integrated with the valuable material, and thus the valuable material is completely incorporated in the body without removal of the valuable material when inserting the microneedles.

Herein, the "integrated" means that there is no definite boundary surface between the original microneedle tip and the newly-adhered part. In the boundary part, the valuable material is considered to have a concentration gradient, because the microneedle is partially dissolved in the aqueous solution of the valuable material. In this case, the composition of the mixture of the water-soluble polymer and saccharides (monosaccharides or disaccharides) to be used for the composition of the microneedle material may be the same as or different from that to be used for the valuable material solution. If they are the same, they are naturally integrated with each other, and even though the water-soluble polymer or the saccharides are different kinds, as long as they are water-soluble, the boundary surface after drying disappears, they are integrated with each other without removal of the valuable material.

### Advantageous Effects of Invention

If the microneedles are dissolved in the skin in a short time, the time for application of the microneedle array to the skin can be considerably reduced. If it is dissolved within 30 minutes, furthermore within 15 minutes, the microneedles can be more easily used and becomes more practical in use at a dermatology office and a beauty parlor for cosmetic application. Also for medical application, the time for administration to a patient can be reduced, and the burden on the patient can be decreased.

Furthermore, if the valuable material is integrally incorporated in the microneedle tips, the valuable material is not removed when inserting the microneedles, and the expensive valuable material can be effectively utilized.

### Brief Description of Drawings

FIG. 1 shows a cross-sectional view in a state that the mold comprising concave portions for forming the microneedles is filled with the aqueous solution of the microneedle material.
FIG. 2 shows a cross-sectional view of the microneedles formed by mold.
FIG. 3 shows shape-comparing views of the microneedles before and after the application.

### Description of Embodiments

Next, Examples of the present invention will be detailed with reference to the figures, but the present invention is not limited to the following Examples.

### [Examples 1-24 and Comparative Examples 1-13]

### (Method for producing microneedle array)

In Examples 1-24 and Comparative Examples 1-13, the microneedle array was produced by using the microneedle materials shown in Table 1 and Table 2 below. Additionally, in Examples 11, 12, 17 and 19, the following valuable materials were further added.

The details of the water-soluble polymers shown in Table 1 and Table 2 are as below.

A hyaluronic acid with molecular weight of 80,000 was obtained from Kikkoman Biochemifa Company (Trade Name: FCH-80LE), a dextran was purchased from Nippon Bulk Yakuhin Co., Ltd. (Trade Name: Dextran 70), a polyvinylpyrrolidone was obtained from BASF Japan Ltd. (Trade Name: KOLLIDON 12PF), a collagen was obtained from Nippi, Incorporated (Trade Name: Rias shark), a ceratin was obtained from Nippi, Incorporated (Trade Name: Nippi High Grade gelatin APAT), and a proteoglycan was obtained from Biomatec Japan,Inc. (Trade Name: Natural proteoglycan).

As saccharides in Table 1 and Table 2, monosaccharides, disaccharides, a kojic acid, an adenosine, a retinoic acid and an α-tocopherol, a sodium chondroitin sulfate, a polyvinyl alcohol, a carboxymethyl cellulose, a hydroxyethyl cellulose were all purchased from Wako Pure Chemical Industries, ltd.

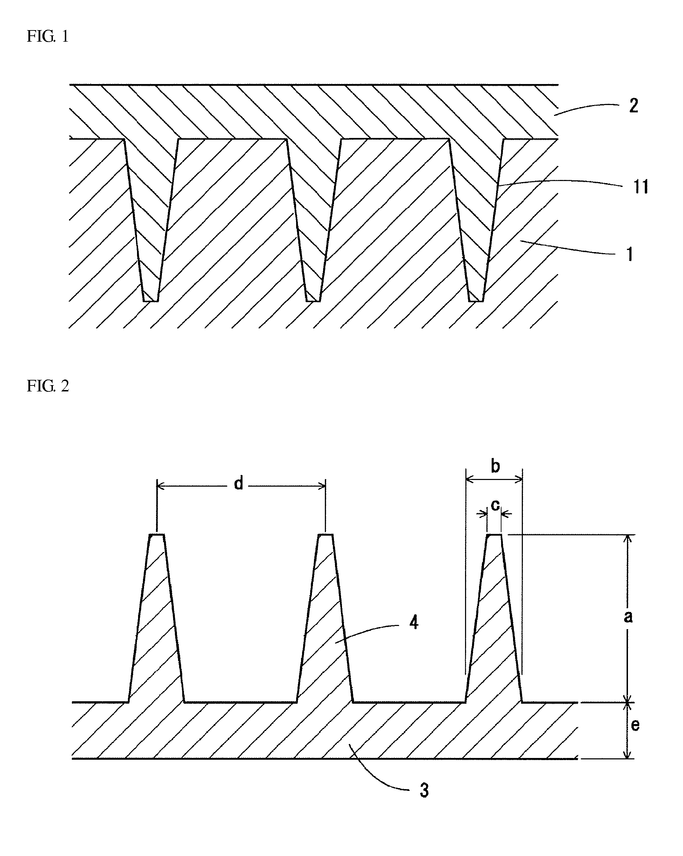

The microneedle array was produced by using the microneedle materials shown in Table 1 and Table 2 and a mold 1 shown in Fig 1 below.

That is, a mold was produced, which comprises a concave portions for forming microneedles, prepared by forming a prescribed shape of microneedle pattern by lithography that light-irradiates a photosensitive resin and then transferring the prescribed shape of microneedle pattern through electro-casting. This mold 1 and the aqueous solution 2 of the microneedle material casted into the upper face of this mold 1 are shown in Fig. 1.

The concave portions 11 for forming microneedles take in a shape of circular truncated cone having 0.2 mm of inlet diameter, 0.04 mm of bottom diameter and 0.8 m of depth, and arranged in a reticular pattern at 0.6 mm intervals, at a rate of 250 portions per 1 cm². In addition, the mold 1 is a square 10 cm on a side.

A cross-sectional shape of the microneedle array formed by this mold 1 is shown in Fig. 2. Each microneedle takes in a shape of circular truncated cone having a needle base diameter b of 0.2 mm, a tip diameter c of 0.04 mm and a height a of 0.8 mm, and is arranged in a reticular pattern with an interval d of 0.6 mm. When the microneedle array is formed in a circle, the microneedles are formed at a rate of about 250 portions per 1 cm². Hereinafter, this shape of the microneedle array will be described as 800-MN.

In the same way, circular truncated cone-shaped microneedles which have a needle base diameter b = 0.4 mm, a tip diameter c = 0.01 mm and a height a = 0.2 mm were produced. This shape of the microneedles are described as 200-MN. Also in this case, the microneedles are formed at a rate of about 250 portions per 1 cm² on the microneedle array.

It should be noted that, in Examples 11, 12, 17 and 19, kojic acid, adenosine, retinoic acid or α-tocopherol was further added as the valuable material as shown in the Table 1 below. First, the mixture of the microneedle material is dissolved in water to prepare an aqueous solution containing 5-20% of solid content. As the valuable material, kojic acid, adenosine, retinoic acid or α-tocopherol was individually dissolved in a small amount of ethanol, added to the aqueous solution of the microneedle material, and mixed. The aqueous solution was casted into the concave portions for forming the microneedles described above at room temperature, dried by vaporizing moisture, and then removed to produce the microneedle array. The microneedle array was cut in a circle with 1 cm of diameter.

The microneedle array was lined with a sheet which was prepared by applying an adhesive on one side of a circler PET with 2 cm of diameter and 16 µm of thickness to produce a microneedle patch.

### (Solubility test for microneedle array in application to humans)

In relation to the combinations of various water-soluble polymers and saccharides, microneedle arrays shown in Tables 1 and 2 were produced. These microneedle arrays were applied to four volunteers. The application of the microneedles means that the microneedles are inserted and held as they are for a certain time. As for the application sites, 200-MN was applied to face, and 800-MN was applied to upper arm. In the case of 200-MN, the microneedle array was removed in 15 minutes, the solubility of the microneedles was microscopically observed. 15 minutes later, even if one out of 4 volunteers showed incomplete dissolution, re-examination was newly conducted, and the solubility in 30 minutes was observed. In the case of 800-MN, the microneedle array was removed in 30 minutes, the solubility of the microneedles was microscopically observed. Whether the dissolution was "complete dissolution" or "incomplete dissolution" was judged by the observation, and ratios of persons who showed complete dissolution out of 4 volunteers were shown in Table 1 as evaluation results. For example, 3/4 means that 3 out of 4 volunteers showed complete dissolution. It should be noted that the "complete dissolution" means that the shapes of the needles are completely dissolved and disappear after the application to the skin, and the "incomplete dissolution" means that the shapes of the needles partially remain.

Table 1 shows the constitutions and the results in Examples, and Table 2 shows constitutions and the results in Comparative Examples. As shown in Table 1, the microneedles of the present invention are completely dissolved within roughly 30 minutes.

It should be noted that, in Tables 1 and 2, the addition amounts of the saccharides are represented by wt% with respect to the total weight of the material of the water-soluble polymer + saccharide. In relation to the valuable materials, K is kojic acid, A is adenosine, R is retinoic acid, V is α-tocopherol, and their addition amounts are represented by wt% with respect to the total weight of the material (water-soluble polymer + saccharide). In addition, * means that a preferable microneedle array could not be produced and skin application test could not be carried out.

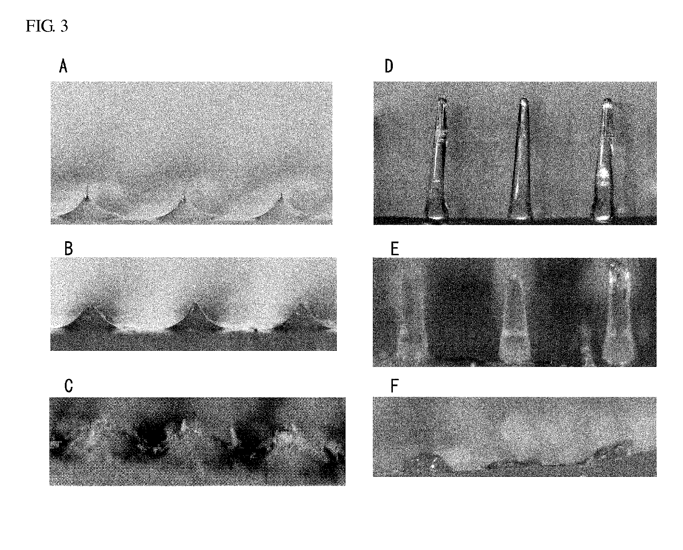

The comparison between the shapes of the microneedles before and after application is shown in Fig. 3. A is a photograph before the application of the microneedle 200-MN to the skin. B is a photograph in the case of the "incomplete dissolution" after the application of 200-MN to the skin. C is a photograph in the case of the "complete dissolution" after the application of 200-MN to the skin. D is a photograph before the application of the microneedle 800-MN to the skin. E is a photograph in the case of the "incomplete dissolution" after the application of 800-MN to the skin. F is a photograph in the case of the "complete dissolution" after the application of 800-MN to the skin.

**[Table 1]**

| Examples of solubility test for the microneedle array in application to humans | | | | | | | | |
|---|---|---|---|---|---|---|---|---|
| Example No. | Microneedle array | | | | | | Evaluation result | |
| | Microneedle material | | | Shape | Valuable material | | | |
| | Water-soluble polymer | Saccharide | | | Name | Addition amount (%) | 15 min. application | 30 min. application |
| | | Name | Addition amount | | | | | |
| Example 1 | hyaluronic acid | glucose | 5 | 200-MN | - | - | 2/4 | 4/4 |
| Example 2 | hyaluronic acid | glucose | 10 | 200-MN | - | - | 3/4 | 4/4 |
| Example 3 | hyaluronic acid | glucose | 15 | 200-MN | - | - | 4/4 | - |
| Example 4 | hyaluronic acid | glucose | 20 | 200-MN | - | - | 4/4 | - |
| Example 5 | dextran | sucrose | 15 | 200-MN | - | - | 2/4 | 4/4 |
| Example 6 | dextran | fructose | 15 | 200-MN | - | - | 3/4 | 4/4 |
| Example 7 | polyvinylpyrrolid one | glucose | 15 | 200-MN | - | - | 3/4 | 4/4 |
| Example 8 | polyvinylpyrrolid one | fructose | 30 | 200-MN | - | - | 4/4 | - |
| Example 9 | hyaluronic acid + collagen (60:40 weight ratio) | glucose | 15 | 200-MN | - | - | 4/4 | - |
| Example 10 | hyaluronic acid + collagen (60:40 weight ratio) | fructose | 30 | 200-MN | - | - | 4/4 | - |
| Example 11 | dextran | sucrose | 30 | 200-MN | K | 1 | 4/4 | - |
| Example 12 | hyaluronic acid | glucose | 30 | 200-MN | A | 0.1 | 4/4 | - |
| Example 13 | hyaluronic acid | glucose | 5 | 800-MN | - | - | - | 2/4 |
| Example 14 | hyaluronic acid | glucose | 15 | 800-MN | - | - | - | 3/4 |
| Example 15 | hyaluronic acid | glucose | 20 | 800-MN | - | - | - | 4/4 |
| Example 16 | hyaluronic acid | glucose | 30 | 800-MN | - | - | - | 4/4 |
| Example 17 | hyaluronic acid | sucrose | 40 | | R | 0.4 | - | 4/4 |
| Example 18 | hyaluronic acid | fructose | 30 | 800-MN | - | - | - | 4/4 |
| Example 19 | hyaluronic acid | glucose + sucrose (15: 15) | 30 | 800-MN | V | 1 | - | 4/4 |
| Example 20 | sodium chondroitin | glucose | 20 | 800-MN | - | - | - | 4/4 |
| | sulfate | | | | | | | |
| Example 21 | proteoglycan | glucose | 30 | 800-MN | - | - | - | 4/4 |
| Example 22 | carboxymethyl cellulose | glucose | 30 | 800-MN | - | - | - | 4/4 |
| Example 23 | hydroxyethyl cellulose | glucose | 30 | 800-MN | - | - | - | 4/4 |
| Example 24 | polyvinyl alcohol | glucose | 30 | 800-MN | - | - | - | 4/4 |

**[Table 2]**

| Comparative Examples of solubility test for the microneedle array in application to humans | | | | | | | | |
|---|---|---|---|---|---|---|---|---|
| Comparative Example No. | Microneedle array | | | | | | Evaluation result | |
| | Microneedle material | | | Shape | Valuable material | | | |
| | Water-soluble polymer | Saccharide | | | Name | Addition amount (%) | 15 min. application | 30 min. application |
| | | Name | Addition amount | | | | | |
| Comparative Example 1 | hyaluronic acid | - | 0 | 200-MN | - | - | 0/4 | 2/4 |
| Comparative Example 2 | hyaluronic acid | - | 0 | 800-MN | - | - | - | 0/4 |
| Comparative Example 3 | hyaluronic acid | glucose | 2 | 800-MN | - | - | - | 0/4 |
| Comparative Example 4 | polyvinylpyrrolido ne | sucrose | 85 | 200-MN | - | - | * | - |
| Comparative Example 5 | dextran | maltose | 90 | 800-MN | - | - | * | - |
| Comparative Example 6 | - | glucose | 100 | 200-MN | - | - | * | - |
| Comparative Example 7 | sodium chondroitin sulfate | - | 0 | 800-MN | - | - | - | 0/4 |
| Comparative Example 8 | proteoglycan | - | 0 | 800-MN | - | - | - | 0/4 |
| Comparative Example 9 | carboxymethyl cellulose | - | 0 | 800-MN | - | - | - | 0/4 |
| Comparative Example 10 | hydroxyethyl cellulose | - | 0 | 800-MN | - | - | - | 0/4 |
| Comparative Example 11 | polyvinyl alcohol | - | 0 | 800-MN | - | - | - | 0/4 |
| Comparative Example 12 | hydroxyethyl cellulose | sucrose | 60 | 800-MN | - | - | * | |
| Comparative Example 13 | hydroxyethyl cellulose | sucrose | 50 | 800-MN | - | - | * | |

### (Method for loading the valuable material into the short-time soluble microneedle)

Bovine insulin (NACALAI TESQUE, INC.) was dissolved in a hydrochloric acid aqueous solution pH 2.5, and this aqueous solution is mixed with an aqueous solution containing 8% of hyaluronic acid (FCH-80LE) and 2% of glucose to produce a valuable material aqueous solution containing the valuable material at a concentration of 1.0 unit (U)/ml to the bovine insulin.

On the microneedle array with a needle length of 800 µm produced in the same way as in Example 17, the microneedle tip portion of 300 µm was soaked in the aqueous solution of the valuable material, immediately pulled out, and dried. The content of the bovine insulin per the microneedle array was 0.08 unit. Hereinafter, this will be abbreviated as a bovine insulin-containing microneedle array A.

Bovine insulin (NACALAI TESQUE, INC.) was dissolved in a hydrochloric acid aqueous solution pH 2.5, and this aqueous solution is mixed with an aqueous solution containing 7% of hydroxyethyl cellulose and 3% of glucose to produce a valuable material aqueous solution containing the valuable material at a concentration of 1.0 unit (U)/ml to the bovine insulin.

On the microneedle array with a needle length of 800 µm produced in the same way as in Example 26, the microneedle tip portion of 250 µm was soaked in the valuable material aqueous solution, immediately pulled out, and dried. The content of the bovine insulin per the microneedle array was 0.07 unit. Hereinafter, this will be abbreviated as a bovine insulin-containing microneedle array B.

Ovalbumin (NACALAI TESQUE, INC.) was dissolved in a phosphate buffer pH 7.5, and this aqueous solution is mixed with an aqueous solution containing 8% of hyaluronic acid (FCH-80LE) and 2% of trehalose (Hayashibara Co., Ltd) to produce a valuable material aqueous solution containing the valuable material at a concentration of 100 µg/ml to the ovalbumin.

On the microneedle array with a needle length of 800 µm produced in the same way as in Example 26, the microneedle tip portion of 200 µm was soaked in the valuable material aqueous solution, immediately pulled out, and dried. The content of the ovalbumin per the microneedle array was 6 µg. Hereinafter, this will be abbreviated as an ovalbumin -containing microneedle array B.

Three male Wistar rats were used and their abdomens were shaved under anesthesia. Using a bovine insulin-containing microneedle array A, a bovine insulin containing-microneedle array B and an ovalbumin-containing microneedle array for each rat, transdermal administration was carried out on abdominal skins of the rats by a spring-type administration assistive equipment. During the administration, the microneedle arrays were fixed by using an adhesive tape. 30 minutes later, the microneedle array was pulled out and microscopically observed, thus the needles were completely dissolved in the skins in all cases.

## Claims

1. A microneedle **characterized by** using, as a microneedle material, a mixture of a water-soluble polymer and one or more saccharides selected from monosaccharides and disaccharides.

2. The microneedle according to Claim 1, wherein a content of the saccharides in the microneedle material is 5-40 wt% with respect to the total weight of the material.

3. The microneedle according to Claim 1 or 2, wherein the microneedle is dissolved within 30 minutes after insertion.

4. The microneedle according to any one of Claims 1 to 3, wherein the saccharides are one or more kinds of saccharides selected from groups comprising glucose, fructose, sucrose, lactose, trehalose, and a mixture thereof.

5. The microneedle according to Claim 4, wherein the saccharide is glucose.

6. The microneedle according to any one of Claims 1 to 3, wherein the water-soluble polymer is a systhetic polymer, protein, polysaccharide or a mixture thereof.

7. The microneedle according to Claim 6, wherein the water-soluble polymer is any one of polyvinylpyrrolidone, polyvinyl alcohol, collagen, gelatin, hyaluronic acid (sodium), dextrin, dextran, proteoglycans, sodium chondroitin sulfate, carboxymethyl cellulose and hydroxyethyl cellulose, or a mixture thereof.

8. The microneedle according to Claim 1, wherein, when adhering the valuable material to the tip of the microneedle of the microneedle array, the valuable material is dissolved in the mixture aqueous solution of the water-soluble polymer and the one or more saccharides in order to produce a valuable material aqueous solution, and then the tip of the microneedle is soaked into the valuable material aqueous solution in order to hold the valuable material integrally with the microneedle tip.

9. A method for short-time soluble of a microneedle array, using a microneedle comprising, as a material, a mixture of a water-soluble polymer and glucose-based monosaccharides, disaccharides or both of them.
